# EUROPÄISCHE PATENTANMELDUNG

(11) **EP 1 076 051 A2**
(43) Veröffentlichungstag der Anmeldung: **14.02.2001**
(21) Anmeldenummer: 00810597.5
(22) Anmeldetag: 07.07.2000
(51) Int. Cl.: C05F 17/02, B65G 65/00, C12M 1/00

(54) **Vergärungsanlage mit Vorrichtung zur Materialaufbereitung**

(30) Priorität: 10.08.1999 CH 146599
(71) Anmelder: Walter Schmid AG, 8152 Glattbrugg (CH)
(72) Erfinder: Kern, Daniel, 8302 Kloten (CH)
(74) Vertreter: Patentanwaltsbüro Feldmann AG

(57) **Zusammenfassung**

Ein Fermenter wird über eine Speisevorrichtung (2) beschickt. Die Speisevorrichtung (2) umfasst eine Mischkammer (23) mit zwei parallelen Schneckenförderern (24) und einer Einfüllöffnung (20), durch welche Frischgut über ein optionales Sieb (21) eingetragen werden kann. Unterhalb der Mischkammer (23) ist ein Förderzylinder (28) angeordnet, der mittels eines Hydraulikzylinders (27) betätigbar ist. Zwischen der Mischkammer (23) und dem Förderzylinder (28) ist ein Verbindungsstutzen (29) angeordnet, der mittels eines Schiebers (34) geöffnet und geschlossen werden kann. Die Verbindungsleitung (33) steht einerseits mit einer Fermenterrückleitung (30), die oberhalb einer tiefsten Stelle des Fermenters ansetzt, und andererseits mit einer Speiseleitung (12), die zum Fermenter (1) führt, in Verbindung. Auch diese Leitungen sind mit entsprechenden Schiebern (31, 32) ausgerüstet.

Die Vorrichtung erlaubt es, Frischgut und weitgehend sedimentfreies Impfgut mittels einer einzigen Pump/Presseinheit (27, 28) in den Fermenter einzuspeisen.

## Beschreibung

Die vorliegende Erfindung betrifft eine vorzugsweise kontinuierlich oder quasi kontinuierlich arbeitende Anlage zum Fermentieren beziehungsweise Vergären von biogenen Abfällen, bei der Frischgut und ein Anteil fermentiertes Gut aus dem Fermenter über eine Speiseleitung eingangsseitig in den Fermenter gelangen.

Anlagen der eingangs genannten Art haben sich ausserordentlich bewährt. Neben einer ökologischen Abfallbewirtschaftung werden mit solchen Anlagen bedeutende Mengen von Biogas erzeugt, die einerseits über BHKWs zum Betrieb der Anlage eingesetzt weraen können und deren Energieüberschuss andererseits entweder in ein Erdgasnetz oder für Antriebszwecke von Motorfahrzeugen Verwendung finden kann.

Solche Anlagen stehen immer auch in Konkurrenz mit anderen Abfallbewirtschaftungsmethoden. So stehen beispielsweise solche Anlagen in Konkurrenz mit Kehrrichtsverbrennungsanlagen. Kehrrichtsverbrennungsanlagen sind zwar weder ökologisch noch ökonomisch, doch eben vielfach bereits bestehend. Sie wurden konzipiert zu einer Zeit, als die getrennte Abfallsammlung in verschiedene Fraktionen kaum üblich war. Entsprechend sind diese Anlagen für grosse Kapazitäten konzipiert und besitzen heute wegen der getrennten Abfallbewirtschaftung Überkapazitäten. Entsprechend nehmen Kehrrichtsverbrennungsanlagen heute Haushaltmüll zu extrem tiefen Preisen entgegen, was der ökologisch vernünftigen getrennten Abfallsammlung und Entsorgung entgegen wirkt.

Zum weiteren stehen die eingangs genannten Anlagen zum Fermentieren biogener Abfälle auch mit den erheblich einfacheren Abfallrotten in Konkurrenz. Die biogenen Abfälle werden hier offen, also aerob, abgebaut. Anlagemässig brauchen solche Rotten nur einen geringen Aufwand, müssen doch lediglich die verrottenden biogenen Abfälle langsam umgeschichtet werden, was mit einfachsten, auf dem Markt erhältlichen Maschinen geschehen kann. Die hierbei freisetzenden Biogase sind allerdings dabei verloren und belasten die Atmosphäre. Praktisch der einzige Kostenpunkt stellt das relativ grosse Areal dar, welches für solche Anlagen benötigt wird. Zudem treten relativ häufig Emissionen auf, die es verunmöglichen, solche Anlagen direkt in Siedlungsgebieten oder an Siedlungsgebiete grenzend zu erstellen.

Viel aufwendiger als die Verrottungsanlagen sind die Anlagen, in denen biogene Abfälle fermentiert beziehungsweise vergärt werden. Die Vergärung erfolgt hierbei anaerob, was relativ grosse, geschlossene Fermenter erfordert.

Bei den erwähnten, von der Anmelderin weltweit erstellten Anlagen wird bis heute so gearbeitet, dass die biogenen Abfälle in einen Annahmebunker gefüllt wurden und von dort über einen Dosierer unter Zwischenschaltung einer Sortieranlage in einen Zerkleinerer gelangte, von dem aus über feste Schnecken- oder Bandförderer das zerkleinerte Biogut in ein Zwischenlager gelangte. Aus dem Zwischenlager wurde das Material direkt oder indirekt entnommen und via einem Wärmetauscher dem Fermenter oder Gärreaktor zugeführt.

Die Eintragung des Frischgutes erfolgte bei bekannten Anlagen, indem das angelieferte Frischgut zerkleinert in einem Bunker zwischengelagert wurde, aus diesem entnommen und mittels einem entsprechenden Fördersystem in die Speiseleitung des Fermenters oder Gärreaktors eingetragen wurde. Das Frischgut weist anfänglich, das heisst bei der Eintragung in den Fermenter eine kaum wesentliche bakterielle Belastung auf, die zum biologischen Abbau der biogenen Abfälle beitragen kann. Um den gesamten Abbauprozess folglich zu beschleunigen, müssen entsprechende Bakterienkulturen zugeführt werden. Dies geschieht durch das Beifügen von abgebautem Material, dem Digestat, welches den Fermenter verlässt.

In der CH-A-682'746 sind zwei verschiedene Varianten solcher Anlagen dargestellt. In einer Variante wird das aus dem Fermenter ausgestossene Material mittels einer Förder-Pressvorrichtung in einer Austragseinheit einerseits ausgetragen und andererseits gepresst. Die Pressung dient dazu, einen Kompost zu gewinnen, mit einem möglichst hohen Trockensubstanzanteil. In der erwähnten Patentschrift wird in einer ersten Version dargestellt und beschrieben, wie mittels der einzigen Press- und Fördervorrichtung nicht nur Frischgut in den Fermenter eingeleitet werden kann, sondern wie mit Hilfe von entsprechenden Steuerklappen, Schieber und Zylinderkolbeneinheiten auch bereits abgebautes, aus dem Fermenter an einer tiefsten Stelle austretendes Material wieder in die Speiseleitung eingebracht werden kann, um so den Fermenter eingangsseitig wieder mit bakteriell stark belastetem Material zu speisen. In der beschriebenen Anlage ist es nicht möglich zwischen Gärprodukt das ausgetragen und der Nachrotte zugeführt werden soll und Gärprodukt das als Impfmaterial in den Fermenter zurückgeführt werden soll zu unterscheiden. Da das Gärprodukt an einer tiefsten Stelle aus dem Fermenter tritt, wird zwangsläufig alles sedimentierte Material, wie Sand, Kies, Steine, Knochen und Aehnliches beim Animpfen dem Fermenter wieder zugeführt. Wie im Weiteren noch ausgeführt wird, bringt dies verschiedene Nachteile mit sich.

Da in der beschriebenen Anlage Gärgut Austrag und Frischgut Eintrag über den selben Förderschacht ablaufen kann nicht sichergestellt werden, dass das hygienisch einwandfreie vergorene Material nicht mit Frischgut kontaminiert wird. So können zum Beispiel keimfähige Samen von Unkräutern aus Frischmaterialresten, welche im Förderschacht verblieben sind, mit nachfolgendem Gärprodukt ausgetragen werden und dieses kontaminieren. Schlimmstenfalls kann das Frischgut Keime enthalten, die Pflanzen- oder Tierkrankheiten hervorrufen können. Die kommerzielle Weiterverwertung des Gärproduktes ist lediglich gewährleistet, wenn dessen hygienische Unbedenklichkeit 100%-ig gegeben ist.

In der zweiten Variante gemäss der CH-A-682'746 sind zum Animpfen und zum Beschicken mit Frischmaterial je eine eigene Förderpumpeinheit nötig. Während die eine Pumpeinheit das auszutragende Material fördert, respektive wiederum mittels Klappen und Schleusen ausgetragenes Material zum Eingang des Fermenters zurückspeist, wird mittels einer zweiten Kolbenzylindereinheit Frischgut in die Speiseleitung gepresst. Hierdurch wird zwar eine kontamination des Gärproduktes mit Frischgut weitgehend Vermeiden, allerdings kann dies nur unter erheblichem Mehraufwand an Infrastruktur erreicht werden.

Neben den verfahrenstechnischen Problemen haben die bekannten Anlagen des weiteren das Problem, dass sie durch die entsprechend langen Förderwege und die Vielzahl an Leitungen und Schleusen beziehungsweise wegen der Verwendung von zwei Pumpeinheiten, kostenintensiv sind.

Es ist daher die Aufgabe der vorliegenden Erfindung, eine Speisevorrichtung zu schaffen, die zur Speisung von Anlagen zum Fermentieren biogener Abfälle gemäss Oberbegriff des Patentanspruches 1 geeignet ist und die bisherigen Nachteile meidet.

Diese Aufgabe löst eine Anlage mit den Merkmalen des Patentanspruches 1.

Im folgenden wird ausgeführt werden, warum es vorteilhaft ist, das Impfmaterial aus dem Fermenter direkt zu entnehmen und nicht aus der Austragsleitung. Wie bereits oben erwähnt, ist es erwünscht unvergärbare Feststoffe, die dem Fermenter mit dem Frischgut zugeführt werden nach einem Durchlauf durch den Fermenter auszutragen. Die Trennung von Gärgut Austrag und Impfmaterialentnahme ermöglicht es Impfmaterial oberhalb eines tiefsten Punktes des Fermenters zu entnehmen. Das direkt dem Fermenter entnommene Material liegt in einer annähernd optimalen Konsistenz vor ist mit aktiven Mikroorganismen beladenenund annähernd frei von unerwünschten Sedimentanteilen ist. Da das Impfmaterial zurück in den Fermenter gespeist wird, wird somit verhindert, dass biologisch inaktiven Sedimentanteile den Fermenter mehrmals durchlaufen, unnötig Volumen einnehmen und im Laufe der Zeit zu unerwünschten Akkumulationen führen. Die Trennung erlaubt bei der Konstruktion des Gärgutaustrages viel mehr Freiheit, da keine Rücksicht mehr auf die Impfmaterialentnahme genommen werden muss.

In der Zeichnung ist eine bevorzugte Ausführungsform des Erfindungsgegenstandes dargestellt, wobei nur die für die Erfindung wesentlichen Funktionsanteile der gesamten Anlage dargestellt sind. Alle Bereiche, die der Biogasgewinnung und Aufbereitung, der Steuerung und der Überwachung dienen sowie dem Handling der Materialzuführung beziehungsweise der Nachbehandlung des Digestates sind hier weggelassen.

Es zeigt:
- Figur 1: eine schematische Darstellung der Einbindung der erfindungsgemässen Speisevorrichtung in der Fermentieranlage und
- Figur 2: die Speisevorrichtung für sich allein in der Seitenansicht in einem grösseren Massstab.

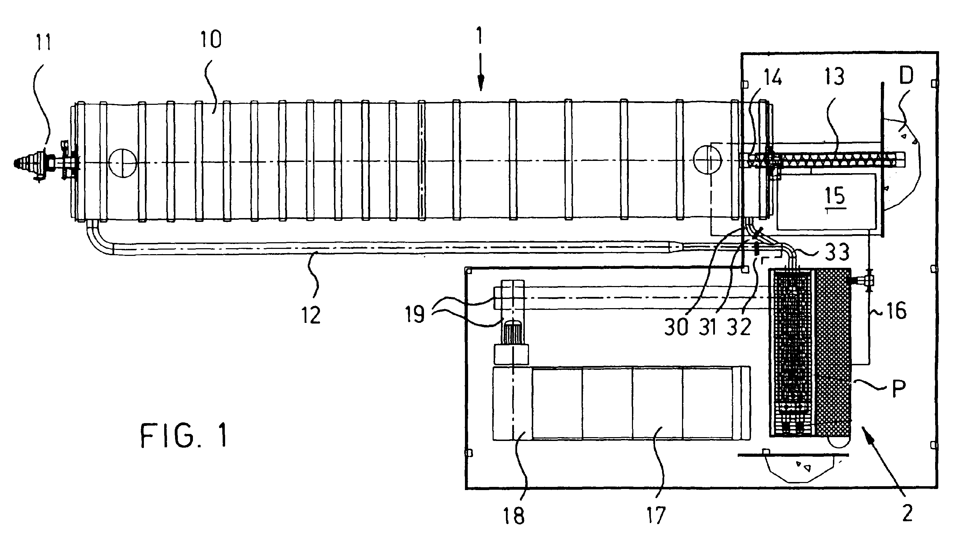

Anhand der Figur 1 werden die Anteile der Anlage zum Fermentieren von biogenen Abfällen erläutert, die für die Erfindung von Bedeutung sind. Der eigentliche Fermenter oder Gärreaktor ist gesamthaft mit 1 bezeichnet. Dieser besteht zur Hauptsache aus dem Fermentertank 10, der entweder als Stahlkonstruktion oder als ausgekleidete Betonkonstruktion gestaltet sein kann. Der Fermentertank 10 wird axial durch ein Rührwerk durchsetzt, welches von einem Rührwerkantrieb 11 getrieben ist. Der Rührwerkantrieb 11 befindet sich an jenem Ende des Fermentertankes 10, in den das zu fermentierende Gut eingespiesen wird. Die Einspeisung erfolgt über die vorzugsweise beheizbare Speiseleitung 12. Die Speiseleitung 12 steht mit der Speisevorrichtung 2 in Verbindung. Von der Speisevorrichtung 2 wird entsprechend aufbereitetes Material durch die Speiseleitung 12 dem Fermentertank 10 zugeführt.

Die Zuführung erfolgt kontinuierlich beziehungsweise quasi kontinuierlich und bestimmt die Durchlaufzeit des zu fermentierenden Materials im Fermenter. Der Durchlauf durch den Fermenter erfolgt folglich ebenfalls kontinuierlich oder quasi kontinuierlich. Von der Austragsöffnung 14 am tiefsten Punkt des Fermenters gelangt das abgebaute, fermentierte Material, das Gärprodukt oder Gärgut, in eine Austragsvorrichtung 13, die hier als kombinierte Austrags-und Entwässerungsvorrichtung gestaltet ist. Das ausgetragene, weitgehend entwässerte Digestat D kann anschliessend einer Nachrotte zugeführt werden. Das mittels der kombinierten Austrags-Entwässerungsvorrichtung 13 gewonnene Presswasser wird in einen Wassertank 15 aufgenommen. Vom Tank 15 kann Verdünnungswasser über eine Leitung 16 in die noch zu beschreibende Speisevorrichtung 2 geführt werden. Als Verdünnungswasser kann Presswasser, sonstiges in der Anlage anfallendes Brauchwasser oder Frischwasser eingesetzt werden.

Die Speisevorrichtung 2 wird hier nicht wie üblich von einem gebäudeintern angeordneten Zwischenbunker gespiesen, sondern das Frischgut gelangt direkt von der Anlieferung auf einen Dosierförderer 17 mit Austragseinheit 18 auf Förderbänder 19, die das Frischgut der Speisevorrichtung zuführen. Ueblicherweise muss das angelieferte Frischgut zerkleinert werden, bevor es dem Fermenter zugeführt werden kann. Dazu können Zerkleinerer eingesetzt werden, die hier nicht dargestellt sind. Statt das Biomaterial auf eine geeignete Grösse zu zerkleinern kann es auch durch Sieben, zum Beispiel in einem Trommelsieb, von zu grossen Bestandteilen befreit werden.

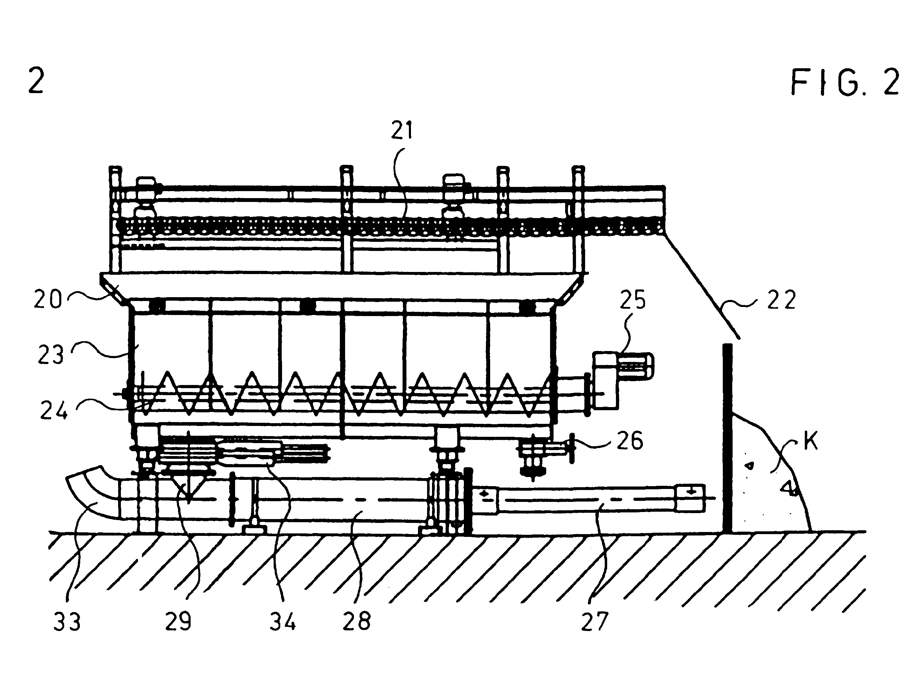

In der Figur 2 ist eine vorteilhafte Ausführungsform der Speisevorrichtung 2 in einer Seitenansicht vereinfacht dargestellt. Mit 20 ist die Einfüllöffnung bezeichnet. Darüber befindet sich ein Scheibensieb 21. Seitlich ist eine Podestfläche P für das Bedienungspersonal angeordnet. Die Podestfläche ist in der Figur 1 ersichtlich. Die Ausgestaltung des Siebes 21 kann unterschiedlich gestaltet sein. Anstelle eines Scheibensiebes kann auch ein Sternsieb, ein Trommelsieb oder ein Spannwellensieb eingesetzt werden. Wie bereits vorgängig erwähnt, ist das Sieb nur nötig, wenn das zugeführte Material Grobgut und Fremdkörper enthält, die nicht in den Fermenter eingespeist werden können oder sollen. Diese Grobteile die nicht durch das Sieb 21 hindurch passen, gelangen über den Siebüberlauf 22 mit einer entsprechenden Rutsche auf eine Kippe K, von sie abgeführt werden können. Durch die Einfüllöffnung 20 gelangt das zerkleinerte und gesiebte Frischgut in eine Mischkammer 23. Im unteren Bereich der Mischkammer 23 sind beispielsweise zwei parallele Schnecken 24 angeordnet, die beide gleich- oder entgegengerichtet antreibbar sind. Hierzu dienen die Antriebsmotoren 25. In die Mischkammer 23 kann über Leitung 16 und Wasserzufuhrventil 26 Verdünnungswasser aus dem Tank 15 eingeleitet werden, was aus Figur 1 ersichtlich ist. Das Verdunnungswasser dient einerseits der Einstellung eines gewünschten Trockensubstanzgehaltes und andererseits der einstellung der gewünschten Homogenität des zu vergärenden Frischgutes.

Unterhalb der Mischkammer 23 befindet sich ein Förderzylinder 28, der mit Hilfe eines Hydraulikzylinders 27 bi-direktional arbeiten kann. Der Förderzylinder 28 steht über einen Verbindungsstutzen 29 mit der Mischkammer 23 in Verbindung. Am Verbindungsstutzen 29 ist ein Schieber 34 angeordnet, der es erlaubt die kommunizierende Verbindung zwischen Mischkamer 23 und Förderzylinder 28 zu öffnen und zu schliessen. Die Speisevorrichtung 2 steht über eine Verbindungsleitung 33 einerseits mit der Fermenterrückleitung 30 und andererseits mit der Speiseleitung 12 in kommunizierender Verbindung. Die Fermenterrückleitung 30 lässt sich mittels eines Schiebers 31 öffnen und schliessen. Auch die Speiseleitung 12 lässt sich mittels eines Schiebers 32 öffnen und schliessen.

Nachfolgend sei die Wirkungsweise der erfindungsgemässen Speisevorrichtung 2 kurz beschrieben. Während Schieber 34 geschlossen ist, wird die Mischkammer 23 durch die Einfüllöffnung 20 bis zu einem gewünschten Füllgrad beschickt. Die beiden Schnecken 24 arbeiten während dieser Zeit im Mischbetrieb und vermengen das eingefüllte Frischmaterial. Während dieser Zeit können über das Ventil 26 Press-, Brauch- oder Frischwasser in die Mischkammer 23 eingeleitet und so, wie bereits erwähnt, der gewünschte Trockensubstanzghalt und die gewünschte Homogenität eingestellt werden. Das Frischmaterial ist nun bereit dem Fermenter zugeführt zu werden.

Nun kann der Schieber 34 im Verbindungsstutzen 29 geöffnet, und durch Zurückziehen des Kolbens im Förderzylinder 28 mittels Hydraulikzylinder 27 das vermischte Frischgut über den Verbindungsstutzen 29 aus der Mischkammer 23 in den Förderzylinder 28 gesaugt werden, bis der Kolben des Förderzylinders 28 eine hintere Position erreicht. Von den beiden Schnecken 24 kann das Material vorgängig schon in einen Bereich über dem Verbindungsstutzen gefördert werden sein. Anschliessend wird der Schieber im Verbindungsstutzen 29 geschlossen und das Frischgut wird bei geöffnetem Schieber 32 vom sich vorwärtsbewegenden Kolben des Förderzylinders 28 über Verbindungsleitung 33 in die Speiseleitung 12 geschoben. Der Schieber 32 kann nun geschlossen werden und wahlweise kann eine weitere Portion Frischmaterial oder wie unten ausgeführt Impfmaterial angesaugt werden. Dazu wird Schieber 31 in der Fermenterrückleitung 30 geöffnet und der Kolben im Förderzylinder 28 mittels des Hydraulikzylinders 27 zurückgezogen, so wird Gärgut aus dem Fermenter in den Förderzylinders 28 gesaugt. Gleichzeitig bleiben jedoch sowohl der Schieber 32 an der Speiseleitung 12 als auch der Schieber 34 am Verbindungsstutzen 29 geschlossen. Das Gärgut wird also über die Fermenterrückleitung 30 und die Speisevorrichtung-Verbindungsleitung 33 in den Förderzylinder 28 gefördert. Diese Förderbewegung wird in geringem Masse von dem Eigendruck des Fermenters, der auf der Fermenterrückleitung 30 anliegt unterstützt. Ist der Förderzylinder 28 mit Digestat gefüllt, so wird der Schieber 31 in der Fermenterrückleitung 30 geschlossen. Der Kolben des Förderzylinders 28 wird aus einer hinteren Position nach vorne bewegt. Über die Speisevorrichtung-Verbindungsleitung 33 wird dabei das Impfmaterial in die vorzugsweise beheizte Speiseleitung 12 ausgeschoben. Hierzu muss selbstverständlich wiederum der Schieber 32 an der Speiseleitung 12 geöffnet sein, während der Schieber 31 in der Fermenterrückleitung geschlossen ist. Der Kolben befindet sich am Ende der Ausschubbewegung in einer vorderen Position im Förderzylinder 28.

Wahlweise kann nun wiederum Frischmaterial oder Impfmaterial in den Förderzylinder 28 eingesaugt und anschliessend der Speiseleitung 12 zugeführt werden.

Dank der erfindungsgemässen Vorrichtung lässt sich der Fermenter 1 mit einer einzigen Pumpeinheit sowohl mit Frischmaterial als auch mit Impfmaterial beschicken. Durch die Trennung von Impfmaterialentnahme und Gärproduktaustrag kann einerseits das Impfmaterial frei von unerwünschtem Sediment gehalten werden, und andererseits erlaubt die Trennung ein Höchstmass an Freiheit bei der Konstruktion und beim Betrieb der Austragseinheit. Die Trennung von Frischguteintrag und Gärproduktaustrag verhindert, dass das hygienisch einwandfreie Gärprodukt durch Frischmaterial kontaminiert wird.

## Patentansprüche

1. Anlage, mit der vorzugsweise kontinuierlich oder quasi kontinuierlich biogene Abfälle fermentiert oder vergärt werden, bei der Frischgut und ein Anteil bakteriell beladenes, fermentiertes Gut aus einem Fermenter (1) über eine Speiseleitung (12) eingangsseitig in den Fermenter gelangen, dadurch gekennzeichnet, dass die Anlage eine Speisevorrichtung (2) aufweist, die eine einzige, bi-direktional arbeitende, räumlich von einer Austragseinheit (13) getrennte, Pump-/Presseinheit (27,28), eine Einfüllöffnung (20) für das Frischgut und eine Mischkammer (23) umfasst zur aktiven Durchmischung des Frischgutes, wobei die Pump-/Presseinheit (27, 28) sowohl fermentiertes Gut aus dem Fermenter (1) als auch den Inhalt aus der Mischkammer (23) in die Speiseleitung (12) und in den Fermenter (1) zu fördern vermag.

2. Anlage nach Patentanspruch 1, dadurch gekennzeichnet, dass eine Fermenterrückleitung (30) vom austrittsnahen Ende des Fermenters (1) direkt zur Speisevorrichtung (2) vorhanden ist.

3. Anlage nach Patentanspruch 2, dadurch gekennzeichnet, dass die Fermenterrückleitung (30) oberhalb des tiefsten Punktes des Fermenters (1) aus diesem austritt.

4. Anlage nach Patentanspruch 1, dadurch gekennzeichnet, dass über der Einfüllöffnung (20) der Speisevorrichtung (2) ein Sieb angeordnet ist.

5. Anlage nach Patentanspruch 4, dadurch gekennzeichnet, dass das Sieb als Scheiben-, Stern-, Trommel- oder Spannwellensieb (21) gestaltet ist.

6. Anlage nach Patentanspruch 4, dadurch gekennzeichnet, dass über dem Sieb ein Magnet für Metallabsonderung angeordnet ist.

7. Anlage nach Patentanspruch 1, dadurch gekennzeichnet, dass in der Mischkammer zwei Schnecken (24) angeordnet sind, die sowohl als Mischer als auch als Austragsmittel betreibbar sind.

8. Anlage nach Patentanspruch 1, dadurch gekennzeichnet, dass die Anlage einen Wassertank (15) umfasst, von dem aus Verdünnungswasser über eine Leitung (16) in die Mischkammer (23) beförderbar ist.

9. Anlage nach Patentanspruch 1, dadurch gekennzeichnet, dass die Mischkammer (23) der Speisevorrichtung (2) über einen verschliessbaren Verbindungsstutzen (29) mit einem Förderzylinder (28) in kommunizierender Verbindung steht.

10. Verfahren zum Betrieb einer Fermenteranlage nach einem der Patentansprüche 1 bis 9, dadurch gekennzeichnet, dass in einem Ansaug-/Ausstoss-Zyklus von der Pump-/Presseinheit (27, 28) Frischgut aus der Mischkammer (23) angesaugt und in die Speiseleitung (12) abgegeben wird und in einem anderen zeitlich getrennten Ansaug-/Ausstoss-Zyklus von der selben Pump-/Presseinheit (27, 28) Impfmaterial von einem Punkt oberhalb des tiefsten Punktes des Fermenters aus dem Fermenter (1) angesaugt und in die Speiseleitung (12) abgegeben wird.

11. Verfahren nach Patentanspruch 10, dadurch gekennzeichnet, dass die Ansaug-/Ausstoss-Zyklen in frei wählbarer Abfolge aufeinanderfolgen.

12. Verfahren nach Patentanspruch 10, dadurch gekennzeichnet, dass das der Mischkammer zugeführte Frischgut vorgängig durch bestehende Bauteile einer Bioabfall-Verwertungsanlage gesiebt wird.
